# EUROPEAN PATENT APPLICATION

(11) **EP 0 744 467 A2**
(43) Date of publication of application: **27.11.1996**
(21) Application number: 96105063.0
(22) Date of filing: 29.03.1996
(51) Int. Cl.: C12N 15/51, A61K 39/29, G01N 33/576, C07K 14/18

(54) **Multiple antigenic peptide comprising at least two hepatitis C virus-associated peptides**

(30) Priority: 30.03.1995 JP 73064/95
(71) Applicant: Eisai Co., Ltd., Tokyo 112 (JP); Arima, Terukatsu, Kagoshima-shi, Kagoshima 892 (JP)
(72) Inventor: Aoyama, Muneo, Inashiki-gun, Ibaraki (JP); Obara, Takashi, Tsukuba-shi, Ibaraki (JP); Tohmatsu, Junichi, Tsuchiura-shi, Ibaraki (JP); Sawada, Takashi, Tsukuba-shi, Ibaraki (JP); Arima, Terukatsu, Kagoshima-shi, Kagoshima (JP)
(74) Representative: Hansen, Bernd, Dr. Dipl.-Chem.

(57) **Abstract**

The reagent for assaying an anti-HCV antibody of the present invention comprising, as an antigen for trapping an anti-HCV antibody, a multiple antigenic peptide comprising at least two HCV-associated peptides enables HCV-infected blood which has been undetectable by the conventional anti-HCV detection reagents to be accurately judged to be positive, thus contributing to the prevention of infection with hepatitis C virus (HCV).

## Description

### Backgroud of the Invention

### Field of the Invention

The present invention relates to a multiple antigenic peptide comprising at least two hepatitis C virus-associated peptides, a reagent for assaying an anti-hepatitis C virus antibody using the multiple antigenic peptide and a method for immunochemically assaying an anti-hepatitis C virus antibody using the multiple antigenic peptide.

### Description of the Related Art

Hepatitis C is a serious disease which, once a human being suffers from this disease, is difficult to be completely cured and, with a high possibility, advances to liver cirrhosis and hepatoma with the years. Blood transfusion is believed to be one of the major routes of infection with a hepatitis C virus (HCV) which is a virus causative of hepatitis C. In recent years, Houghton et al. have reported the isolation of the gene of the virus (see European Patent Publication-A No. 318,216) and have commercialized a reagent for detecting the antibody by EIA or other method using a virus antigen. An attempt has been made to detect an anti-hepatitis C virus antibody in serum using this reagent, thereby enabling the exclusion of blood for transfusion containing a hepatitis C virus. Although posttransfusion hepatitis has been reduced thereby, it cannot be thought to be on a negligible level as yet. Further, there are often reported on the detection of a hepatitis C virus RNA (HCV-RNA) from specimens of the patients diagnosed as suffering from non-A, non-B, non-C hepatitis using conventional diagnostic reagents.

Although one may well say that this is because the relationship between the viral infections and the antibody production has not been elucidated yet, it is conceivable as the cause that there is a considerable heterogeneity in the nucleotide sequence of the genome of the virus. This has been substantiated by the results of many experiments on the cloning of the viral genome c-DNA by PCR, primer extension or the like [see Kubo et al., Nucleic Acid Research, 17, 10367-10372 (1989); Kato et al., Proc. Japan Acad., 65B, 219-223 (1989); Maeno et al., Nucleic Acid Research, 18, 2685-2689 (1990); and Kato et al., Proc. Natl. Acad. Sci. USA, 87, 9524-9528 (1990)]. Thus, the diagnosis of the hepatitis C using conventional reagents for detecting an anti-hepatitis C virus antibody and the prevention of hepatitis C attributable to blood transfusion cannot be said to be satisfactory, and the present situation is in that the development of a diagnostic reagent with high detection performance is desired in the art in order to minimize the sideration of hepatitis C.

Interferon therapy is now carried out as the most effective therapy for hepatitis C. However, it is not easy to learn the therapeutic effect and whether or not the hepatitis has been completely cured. At present, the most reliable method therefor comprises detecting the HCV-RNA by RT-PCR over one year. However, it is known that, even in the cases of recurrence, the HCV-RNA content is lowered to a level below the detection limit of the RT-PCR method in many cases. For this reason, when necessary cost and labor are taken into consideration, it is difficult to say that the RT-PCR method is effective. There is also a method wherein the antibody titer of the core antibody is measured to aid the judgment of the therapeutic effect. This method, however, is disadvantageous in that a period of six months or longer is required for judgment and, in many cases, it is difficult to judge the therapeutic effect.

### Disclosure of the Invention

### Summary of the Invention

An object of the present invention is to provide a multiple antigenic peptide comprising at least two hepatitis C virus-associated peptides, which specifically binds to an anti-hepatitis C virus antibody.

Another object of the present invention is to provide a diagnostic reagent which can detect, with a higher percentage of detection, an anti-hepatitis C virus antibody present in a blood of a patient infected with hepatitis C virus and undetectable by the conventional diagnostic reagents for detecting an anti-hepatitis C virus antibody and which is also useful for the judgment of the therapeutic effect on hepatitis C.

The other object of the present invention is to provide a method for immunochemically assaying an anti-hepatitis C virus antibody useful in a clinical field.

At the present time, several amino acid sequences for HCV are known in the art, and examples thereof include HCV-E (see European Patent Publication-A No. 628,572), HCV-1, HCV-J, HCV-J6 and HCV-J8. The prediction as to which portion of the amino acid sequence for HCV corresponds to the epitope site of the antigen is possible by various analytical methods. Examples of these methods include Super Mirror Service (Mitsui Knowledge Industry Co., Ltd.), prediction of secondary structure [see Robson B. and Suzuki E., J. Mol. Biol., 107, 327-356 (1976)], and hydrophilicity profile [see Hopp T. P. and Woods K. R., Proc. Natl. Acad. Sci. USA., 78, 3824-3828 (1981)]. The present inventors have synthesized peptides each comprising the amino acid sequence of the antigenic site predicted by these analytical methods and have made extensive studies on the antigenicity of the peptides. As a result, they have found that a plurality of peptides each comprising the amino acid sequence of the epitope site have strong antigenicities when each peptide is tested in the form of a multiple antigenic peptide comprising at least two peptides each comprising the amino acid sequence of the epitope site, although the peptide comprising the amino acid sequence of the epitope site, alone, i.e., a peptide monomer, exhibits little or no antigenicity. Accordingly, the present inventors have for the first time found that the synthetic multiple antigenic peptide can be used as an antigen for efficiently detecting anti-HCV antibodies. The present invention has been completed on the basis of these findings.

Thus, the present invention provides a multiple antigenic peptide which comprises at least two hepatitis C virus-associated peptides each comprising at least eight consecutive amino acid residues in an amino acid sequence of a protein constituting a hepatitis C virus.

The "multiple antigenic peptide" refers to a compound which is prepared by directly binding a plurality of HCV-associated peptides one another, or by indirectly binding them. Examples of the indirect binding include the binding of them through a material for crosslinkage such as a skeleton and a core matrix. Regarding the mode of the binding, the HCV-associated peptides may be bonded to each other either covalently or noncovalently.

The multiple antigenic peptide is preferably such that a contemplated number of contemplated HCV-associated peptides are bonded to a material for crosslinkage having a plurality of amino groups as the reactive groups. The material for crosslinkage may be any one so far as it has a plurality of groups reactive with an amino acid or a peptide. However, it is preferably a polylysine wherein a contemplated number of lysine are bonded to β-alanine or the like.

The HCV-associated peptides constituting the multiple antigenic peptide of the present invention may be the same or different from one another. Specially, when the multiple antigenic peptide is an antigenic peptide octamer comprising a material for crosslinkage having eight amino groups and eight HCV-associated peptides bonded to the material for crosslinkage, it may be a homo-octamer wherein the eight HCV-associated peptides are the same one another; or a hetero-octamer wherein one species of HCV-associated peptide is bonded to each of the four amino groups of the material for crosslinkage and the other species of HCV-associated peptide is bonded to each of the other four amino groups thereof. When the multiple antigenic peptide according to the present invention is a hetero type, it is necessary that the multiple antigenic peptide comprises two or more of respective species of the HCV-associated peptides. The hetero-type multiple antigenic peptide may be prepared according to a known method, e.g., by binding two or more species of homo-type multiple antigenic peptides one another.

The term "a protein constituting a hepatitis C virus" herein includes equivalents of the HCV constituting protein. The term "equivalent" in the above situation is defined in that it immunochamically acts in a similar manner to that of the HCV constituting protein, and has the similar amino acid sequence to that of the HCV constituting protein except that one or more another amino acid residue(s) is(are) added thereto, inserted thereinto or substituted therefor, or that one or more amino acid residue(s) is(are) deleted therefrom, which brings about a silent alteration to the HCV constituting protein. For example, when amino acid (I) has the similar property, with respect to, e.g., the polarity, electric charge, solubility or hydrophobicity, to that of amino acid (II) in the sequence and amino acid (I) substitutes for amino acid (II), the protein thus prepared is an equivalent.

The present invention includes a hepatitis C virus-associated synthetic peptide multimer which is a multimer (i.e., n-mer wherein n is an integer of 2 or more) of a hepatitis C virus-associated synthetic peptide comprisimg a sequence of at least eight consecutive amino acids in any one of the peptides specified in SEQ ID NOs: 2-13.

Further, the present invention provides a reagent for assaying an anti-hepatitis C virus antibody, which comprises, as an antigen for detecting the anti-hepatitis C virus antibody, at least one multiple antigenic peptide described above.

In other words, the present invention relates to a reagent for assaying an anti-hepatitis C virus antibody, which comprises, as an antigen for detecting an anti-hepatitis C virus antibody, a multimer (i.e. n-mer wherein n is an integer of 2 or more) of a peptide comprising at least eight consecutive amino acids in the amino acid sequence of a protein constituting a hepatitis C virus.

Furthermore, the present invention provides a method for immunochemically assaying an anti-hepatitis C virus antibody, which comprises the steps of:
reacting a specimen sample with a multiple antigenic peptide described above,
reacting the reaction product with a labelled anti-human immunoglobulin antibody, and
determining the amount of the labelled anti-human immunoglobulin antibody bound to the reaction product.

Generally, this method further comprises washing steps.

The specimen sample is desirably a human biological sample.

The multiple antigenic peptide has preferably been immobilized on a carrier such as a solid phase.

The present invention includes a method for immunochemically assaying an anti-hepatitis C virus antibody, comprising the steps of reacting a specimen sample with solid phase onto which a peptide multimer (i.e., n-mer wherein n is an integer of 2 or more) of a peptide comprising at least eight consecutive amino acids in the amino acid sequence of a protein constituting a hepatitis C virus has been immobilized, reacting, after washing, a labelled anti-human immunoglobulin antibody therewith, and determining, after washing, the amount of the bound labelled anti-human immunoglobulin antibody.

The present inventors have found that, in many cases, the use of the reagent for detecting an anti-HCV antibody using, as an antigen, a multiple antigenic peptide according to the present invention enables the detection of an anti-HCV antibody from the serum from which the antibody cannot be detected by the conventional reagents for detecting anti-HCV antibodies, though the serum contains HCV genes. Further, it has been found that the reagent of the present invention can clearly judge the therapeutic effect in the interferon therapy, i.e., that the antibody titer determined with the reagent of the present invention is greatly lowered in the serum of a patient suffering from hepatitis C for whom the interferon therapy is significantly effective while no significant lowering in antibody titer is observed in the serum of a patient suffering from hepatitis C to whom the interferon therapy is ineffective.

Further scope and applicability of the present invention will become apparent from the detailed description and examples given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description and these examples.

### Detailed Description of the Invention

Based on the amino acid sequence of an HCV-constituting protein which has been known, an epitope site is predicted, and a peptide comprising the amino acid sequence of the predicted epitope site is synthesized. A general method for synthesizing a peptide may be used for synthesizing the above peptide, and examples thereof include the activated ester method, the mixed acid anhydride method, the C-terminal activation method such as the azide method, the coupling method using a coupling agent such as carbodiimide, the N-carboxy anhydride (NCA) method, the redox method, the enzymatic method, and the solid phase synthesis method. A multiple antigenic peptide may be synthesized, for example, by Tam's method [see James P. Tam., Proc. Natl. Acad. Sci. USA., 85, 5409-5413 (1988)].

The contemplated material for crosslinkage, i.e. a core matrix, can be prepared by chemically bonding a contemplated number of lysines to a resin via β-alanine, which has been immobilized on the resin, in a stepwise manner according to any conventional synthetic method. Specifically, β-alanine with one lysine bonded thereto may be used as a core matrix for a peptide dimer, β-alanine with three lysines prepared by binding two lysines to the core matrix for a peptide dimer may be used as a core matrix for a peptide tetramer, and β-alanine with seven lysines prepared by binding four lysines to the core matrix for a peptide tetramer may be used as a core matrix for a peptide octamer. By chemically binding amino acids constituting the contemplated HCV-associated peptides in order, to the above material for crosslinkage, i.e., a core matrix, by any conventional method, a multiple antigenic peptide of the present invention can be prepared.

In the present invention, the number of the HCV-associated peptides constititing the multiple antigenic peptide is two or more and is not particularly limited. It, however, is preferably 2 to 8, still more preferably 8, from the practical viewpoint.

Regarding the number of the amino acid residues constituting an antigen peptide for detecting an anti-HCV antibody, it is known that a peptide consisting of six amino acid residues binds to an antibody (see International Publication No. 8403564, published on September 13, 1984; Applicant: COMMONWEALTH SERUM LABS AND GEYSEN, H.M.). Therefore, the number of constitutive amino acid residues of a peptide usable as an antigen is at least 6, preferably at least 8, still more preferably at least 10, most preferably 13 to 20.

Each HCV-associated peptide, i.e., peptide monomer, constituting the multiple antigenic peptide of the present invention comprises at least eight consecutive amino acid residues in an amino acid sequence of a protein constituting a hepatitis C virus. Each HCV-associated peptide preferably comprises at least eight consecutive amino acid residues in an amino acid sequence specified in any of SEQ ID NOs: 1-13; and still more preferably comprises the twelve amino acid residues of SEQ ID NO: 1, the thirteen amino acid residues of any of SEQ ID NOs: 2-10, the fifteen amino acid residues of SEQ ID NO: 11 or 13, or the seventeen amino acid residues of SEQ ID NO: 12.

In the present invention, the HCV-associated peptide constituting the multiple antigenic peptide of the present invention may comprise at least eight consecutive amino acid residues which is immunochemically equivalent to the at least eight consecutive amino acid residues in any of SEQ ID NOs: 1-13. That is, the HCV-associated peptide comprises at least eight consecutive amino acid residues which are similar to the at least eight consecutive amino acid residues in any of SEQ ID NOs: 1-13 except that one or more another amino acid residue(s) is(are) added thereto, inserted thereinto, or substituted therefor, or that one or more amino acid residue(s) is(are) deleted therefrom, which brings about a silent alteration to the amino acid sequence. In such a case, the immunochamical property shown by the at least eight consecutive amino acid residues in any of SEQ ID NOs: 1-13 is maintained.

The MAP (multiple antigenic peptide) method used herein as a method for preparing a synthetic peptide in the multiple form is a method developed by Tam et al. for the purpose of providing an antigen for vaccines capable of enhancing the immunological effect without conjugation with other carrier protein. The present inventors have for the first time found that the above multiple antigenic peptide is usable for the assaying of an anti-HCV antibody, that even a peptide having a weak antigenicity, when brought to a multiple form, exhibits a strong antigenicity to be usable for the assaying of an anti-HCV antibody, and that the above multiple antigenic peptide can exhibit such an excellent effect that specimen samples which have not been judged positive by the previous assaying method can precisely be judged as positive by using the above multiple antigenic peptide. Accordingly, the present invention is characterised in that a multiple antigenic peptide comprising HCV-associated peptides each comprising a partial peptide of proteins constituting various HCVs are used in a reagent for detecting an anti-HCV antibody. The number of the amino acid residues constituting the partial peptide may be the same as that described above, and the partial peptide is composed of at least six amino acid residues, preferably at least eight amino acid residues, still more preferably at least ten amino acid residues, most preferably 13 to 20 amino acid residues.

Conventional methods may be employed for immunochemical assay of an anti-HCV antibody using a multiple antigenic peptide, and examples thereof include ELISA, Western blotting, and agglutination. ELISA is preferred from the viewpoints of convenience and utility. For example, a specimen sample is added to a well onto which one or several species of multiple antigenic peptide(s) have been immobilized to effect a reaction, thereby forming an antigen-antibody complex. The well is washed and then an enzyme-labeled anti-human immunoglobulin antibody is added thereto to react with the complex. After washing, the amount of the bonded enzyme is determined using a coloring substrate. Further, when the properties of the multiple antigenic peptide are taken into consideration, the use of a labeled multiple antigenic peptide instead of the enzyme-labeled anti-human immunoglobulin antibody can be expected.

Labels for the anti-human immunoglobulin antibody or the multiple antigenic peptide include enzymes, fluorescent substances, radioactive substances, bioluminescent substances, chemiluminescent substances and electrochemiluminescent substances. The carrier onto which a multiple antigenic peptide is immobilized may be any one, and examples thereof include microtiter plates, plastic beads, erythrocytes, gelatin particles, latex particles and magnetic particles.

An embodiment of the reagent of the present invention for ELISA will now be described. Specifically, the reagent comprises a multiple antigenic peptide comprising at least two HCV-associated peptides as an indispensable constituent, a carrier (preferably a solid phase), a standard anti-HCV antibody, an enzyme-labeled anti-human immunoglobulin antibody and an enzyme substrate. The reagent may further comprise a diluent for the antibody, a solution for a reaction, a liquid for dissolving a substrate, a liquid for terminating the reaction, a washing liquid, etc. suitable for convenience of practice of the assaying, which does not limit the present invention.

In the reagent of the present invention, the species of the multiple antigenic peptide used as the antigen is not limited to one and, if necessary, a combination of a plurality of species of multiple antigenic peptides may be used as a component of the reagent. Further, the multiple antigenic peptide may be used in combination with a peptide monomer(s). For example, a reagent for detecting an anti-HCV antibody by using, as antigens, a multiple antigenic peptide comprising HCV-associated peptides each having the amino acid sequence specified in SEQ ID NO: 11, another multiple antigenic peptide comprising HCV-associated peptides each having the amino acid sequence specified in SEQ ID NO: 12 and the other multiple antigenic peptide comprising HCV-associated peptides each having the amino acid sequence specified in SEQ ID NO: 13 has been confirmed to have an effect comparable with or superior to the second-generation reagents for detecting an anti-HCV antibody in current clinical use.

The use of the synthetic multiple antigenic peptide of the present invention enables hepatitis C, of which causative virus has been undetectable by anti-HCV antibody assays using conventional antigens, to be diagnosed, contributing to the prevention of infection with the hepatitis C virus through blood transfusion. Further, by the immunochemically assaying method according to the present invention, the prediction and judgment of the effect of the interferon therapy can be carried out in an earlier stage and in a simpler manner than with the prior art method.

### Brief Description of the Drawings

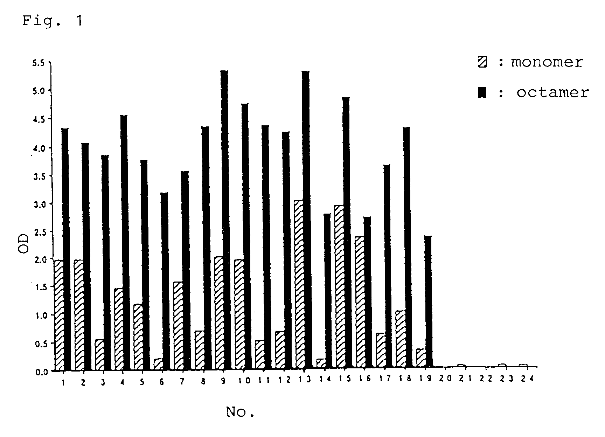

Fig. 1, which shows the effect of the present invention, is a diagram showing a comparison of the sensitivities for the detection of antibody regarding the use of a peptide monomer or a peptide octamer as an antigen, wherein the solid bar refers to a peptide octamer, the hatched bar to a peptide monomer, Nos. 1-19 to plasmas of patients suffering from hepatitis C, and Nos. 20-24 to normal human plasmas.

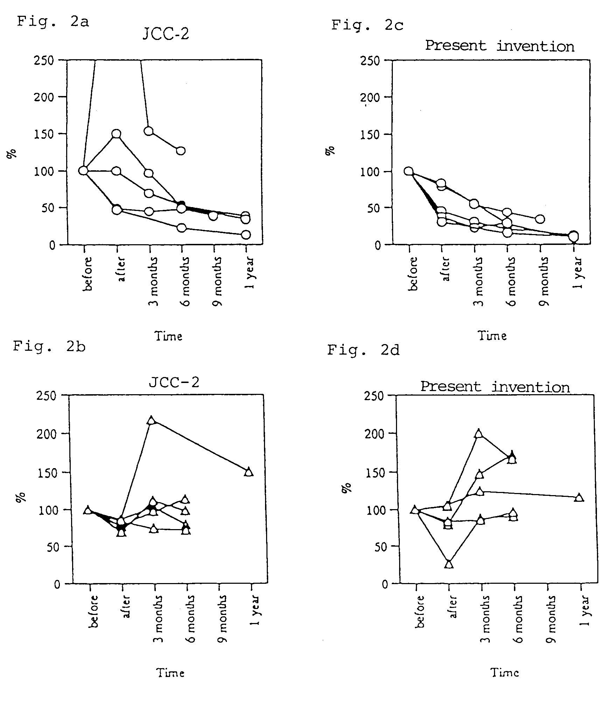

Figs. 2a, 2b, 2c and 2d are diagrams each showing changes in the blood anti-HCV antibody levels of patients suffering from hepatitis C in interferon therapy for cases where the therapy was significantly effective (Figs. 2a and 2c) and another cases where the therapy was ineffective (figs. 2b and 2d), wherein the results of an experiment for the reagent of the present invention are compared with the results of an experiment for Chemo-Sero EIA "JCC-2".

### Examples

The present invention will now be described in more detail with reference to the following examples which should not be considered to limit the scope of the present invention.

### Example 1: Enzyme immunoassay of anti-hepatitis C virus antibody in serum

100 µl of a synthetic peptide (monomer, dimer, tetramer or octamer) solution (5 µg/ml) was poured into each well of a microplate. The microplate was allowed to stand at 25°C overnight and then washed three times with PBS/0.05% Tween 20, thus immobilizing the synthetic peptide onto the wells. 100 µl of a solution for a reaction [100% normal rabbit serum buffered with Tris-HCl (pH 8.0)] was poured into each well of the plate, and 20 µl of the plasma of a subject is poured thereinto. The plate was incubated at 37°C for 60 min, thereby reacting an anti-HCV antibody in the plasma of the subject with the immobilized synthetic peptide. After the wells were washed five times with PBS/0.05% Tween 20, 100 µl of a solution of an anti-human immunoglobulin antibody labeled with an alkali phosphatase was added to each well, and the plate was incubated at 37°C for 60 min, thereby reacting the anti-human immunoglobulin antibody with the anti-HCV antibody immobilized onto the synthetic peptide. After the wells were washed five times with PBS/0.05% Tween 20, 100 µl of a substrate (p-nitrophenyl phosphate) solution (1 mg/ml) was added to each well. The plate was incubated at 37°C for 30 min, and then 100 µl of a 1 N sodium hydroxide solution was added to each well to stop the reaction. The absorbance of the solution in each well at 405 nm was measured.

### Example 2: Detection of anti-hepatitis C virus antibody in serum by electrochemiluminescence (ECL)

1 ml of magnetic beads (Dynabeads M-450; tosyl activated) solution was dehydrated, and 1 ml of a borate buffer (pH 9.5) and 50 µl of a synthetic peptide solution (1 mg/ml) were added thereto. The obtained mixture was stirred at room temperature to bond the synthetic peptide onto the magnetic beads. Thereafter, 1 ml of 0.1 M Tris buffer (pH 8.0) was added thereto, and the resulting mixture was stirred at room temperature for 1 hr. Thereafter, the magnetic beads were washed five times with 50 mM Tris buffer (pH 8.0) and suspended in a concentration of 1 mg/ml in a bead suspending buffer [50 mM Tris-HCl (pH 8.0), 0.01% Tween 20, and 0.1% NaN₃] to prepare a suspension. 200 µl of a solution for a reaction, 25 µl of the suspension of the magnetic beads onto which the synthetic peptide was bonded, and 20 µl of plasma of a subject were added to a polypropylene tube, and a reaction was allowed to proceed at room temperature for 8 min. The magnetic beads were washed three times with a washing liquid [10 mM Tris-HCl (pH 7.5), 0.15 M NaCl, 0.05% NaN₃, and 0.001% Tween 20], 200 µl of an anti-human immunoglobulin monoclonal antibody labeled with ruthenium was added thereto, and a reaction was allowed to proceed at room temperature for 8 min. The magnetic beads were washed three times with a washing liquid, and 300 µl of an assay buffer (0.2 M potassium phosphate (pH 7.5), 0.1 M TPA, 0.5% Triton X100, 0.5% Tween 20, and 0.5% NaN₃) was added thereto. Thereafter, the electrochemiluminescence thereof was measure with a photometer.

### Example 3: Synthesis of multiple antigenic peptide

A multiple antigenic peptide was synthesized according to Tam's method (James P. Tam, Proc. Natl. Acad. Sci. USA, 85, 5409-5413, 1988). Specially, the synthesis of the multiple antigenic peptide was effected as follows: Fmoc-β-Ala-OH in an amount of 3/8 molar equivalent relative to NovaSyn KA 125 resin (0.08 mmol/g, NOVA biochem) was suspended in dichloromethane (DCM). 1,3-Diisopropylcarbodiimide in an amount equimolar with Fmoc-β-Ala-OH was dropwise added to the suspension in ice, and a reaction was allowed to proceed with stirring. The resin swollen with dimethylformamide (DMF) was reacted with Fmoc-β-Ala-OH by stirring a mixture of the resin, the reaction mixture obtained in the previous step and a DMAP (4-dimethylaminopyridine, 1/8 molar equivalent relative to the resin)/DMF solution. After the completion of the reaction, the resin was washed with DCM, methanol and diethyl ether in that order, 10 mg of the resin (in the case of 0.1 mmol/g resin) was treated with 20% solution of piperidine in DMF to remove the protective groups of the resin. The absorbance at 290 nm was then measured, and the percentage dissociation of Fmoc was calculated to examine the percentage incorporation of β-Ala. The reactive groups of the remaining resin were acetylated with a 20% solution of acetic anhydride in DMF, and the protective groups were then removed with a 20% solution of piperidine in DMF. Thus, β-Ala was introduced into the resin.

Thereafter, Lys was introduced thereinto as follows: N-methylmorpholine (NMM) was dropwise added to a DMF solution of Fmoc-Lys (Fmoc)-OH, PYBOP and HoBt in respective amounts of 4 times, 3 times and 3 times the amount of β-Ala introduced into the resin. The introduction of Lys into the resin with β-Ala introduced thereinto was assayed in terms of the percentage dissociation of the Fmoc group. After the introduction of Lys was confirmed, the acetylation and the deprotection were carried out in the same manner as described above in connection with the introduction of β-Ala. The second introduction of Lys (i.e., the formation of 4-branches), the acetylation and the deprotection were carried out in the same manner as that described above. In this case, the amounts of Fmoc-Lys (Fmoc)-OH, PyBOP and HoBt used were respectively 4 times, 3 times and 3 times the amount of Lys introduced in the first introduction thereof (i.e., the formation of 2-branches). The third introduction of Lys (i.e., the formation of 8-branches) was carried out in the same manner as described above to prepare a material for crosslinkage, i.e., a core matrix, usable in the synthesis of a multiple antigenic peptide in octamer form.

By using the resin having a core matrix introduced thereinto prepared as described above, a multiple antigenic peptide in octamer form was prepared as follows: A peptide synthesizer Biolynx 4170 (Pharmacia LKB) was used to remove the Fmoc protective groups of this resin, and amino acids were successively reacted with the amino group derived from Lys by any conventional synthetic method. The core matrix prepared by reacting Lys once was used for the synthesis of a multiple antigenic peptide in dimer form, and the core matrix prepared by reacting Lys twice was used for the synthesis of a multiple antigenic peptide in tetramer form.

### Example 4: Comparison of antibody detection sensitivities using peptide monomer and peptide octamer (by EIA)

A comparative experiment was carried out on the antibody detection sensitivity by EIA using, as an antigen, a monomer of a peptide having the amino acid sequence specified in SEQ ID NO: 11, and an octamer of the peptide. The peptide octamer was synthesized by the method described in Example 3 according to Tam's method.

The results of the detection sensitivity assay are shown in Fig. 1. In Fig. 1, the solid bar represents the results of an experiment using the peptide octamer as the antigen, while the hatched bar represents the results of an experiment using the peptide monomer as the antigen. Nos. 1 to 19 in the abscissa represent plasma specimens of patients suffering from hepatitis C, while Nos. 20 to 24 in the abscissa represent normal human plasma specimens. For all the 19 plasma specimens of patients suffering from hepatitis C, when the peptide octamer was used as the antigen, the color density (OD₄₀₅) was about 1.7 to 20 times higher than that when the peptide monomer was used as the antigen. While, for all the five specimens of normal human sera, when the peptide octamer was used as the antigen, the color density (OD₄₀₅) was lower than that when the peptide monomer was used as the antigen.

### Example 5: Detection of anti-hepatitis C virus antibody in serum using monomer, dimer, tetramer and octamer of synthetic peptide as antigen

Specimens which were HCV-RNA positive but were negative for the second-generation antibody detection reagents were subjected to EIA using, as antigens, a monomer, a dimer, a tetramer and an octamer of a peptide having the amino acid sequence specified in SEQ ID NO: 2. As described in Example 3, regarding the multiple antigenic peptides, the peptide tetramer could be synthesized using β-alanine with three lysine residues bonded thereto as the core matrix, and the peptide dimer could be synthesized using β-alanine with one lysine residue bonded thereto as the core matrix.

As is apparent from the results given in Table 1, no reaction took place for the peptide monomer, whereas the reactivity to an anti-HCV antibody increased in order of the peptide dimer, peptide tetramer and peptide octamer. Further, the core matrix of the multiple antigenic peptide alone did not react with any specimen sample. This shows that an anti-HCV antibody can be detected only when a peptide is used in the multiple form, which enables HCV-infected specimen samples that have been undetectable by the conventional methods to be detected, contributing a reduction in infection with HCV through blood transfusion.

**Table 1**

| | OD₄₀₅ | | | | |
|---|---|---|---|---|---|
| Sample | Monomer | Dimer | Tetramer | Octamer | Core Matrix |
| III-13 | 0.055 | 2.454 | 3.722 | 3.431 | 0.055 |
| 128 | 0.071 | 3.679 | 3.459 | 4.000 | 0.076 |
| 134 | 0.069 | 0.717 | 2.445 | 3.070 | 0.071 |
| 142 | 0.090 | 0.152 | 2.676 | 3.248 | 0.080 |
| 144 | 0.070 | 0.784 | 2.472 | 3.448 | 0.072 |
| 145 | 0.073 | 0.227 | 1.379 | 2.398 | 0.061 |
| NHS | 0.061 | 0.057 | 0.074 | 0.107 | 0.060 |
| Blank | 0.047 | 0.050 | 0.063 | 0.062 | 0.054 |
| PC | 0.082 | 0.083 | 2.739 | 3.280 | 0.070 |
| Note) NHS: normal human serum, and PC: positive control. | | | | | |

### Example 6: Comparison of antibody detection sensitivity using multiple antigenic peptide (by ECLIA)

The peptides specified in SEQ ID NOs: 11, 12 and 13 were used as monomer peptides, and a comparative experiment was carried out on the antibody detection sensitivity of the peptide monomers and peptide octamers as antigens by ECLIA. The peptide octamers were synthesized by the method described in Example 3 according to Tam's method.

The results of the detection sensitivity assay are given in Table 2. The peptide monomers reacted scarcely or only little with the specimen samples positive for anti-HCV antibody, whereas the peptide octamers had very high reactivity with the specimen samples, i.e., high antigenicity. Further, the peptide octamers were found to be unreactive with normal human sera.

**Table 2**

| | Peptide of SEQ ID NO: 11 | | Peptide of SEQ ID NO: 12 | | Peptide of SEQ ID NO: 13 | |
|---|---|---|---|---|---|---|
| | monoer | octamer | monomer | octamer | monomer | octamer |
| Sample positive for anti-HCV antibody (not diluted) | 1208 | 328355 | 16429 | 155930 | 1540 | 496156 |
| Sample positive for anti-HCV antibody (diluted to 1/10 conc.) | 257 | 50288 | 1961 | 13862 | 241 | 68418 |
| Normal human serum | 1844 | 3875 | 1873 | 196 | 1951 | 210 |
| Blank | 96 | 119 | 117 | 130 | 109 | 113 |

### Example 7: Assay of specimen samples negative for second-generation anti-HCV antibody detection reagent

Twenty-two specimen samples of sera which were positive for HCV-RNA but were negative for all of HCV-PHA "Dainabot" (manufactured by Dainabot Co., Ltd.), Immucheck HCV Ab (manufactured by International Reagents Co., Ltd.), and Synpep HCV-EIA (manufactured by Kyokuto Pharmaceutical Industrial Co., Ltd.) as the second-generation anti-HCV antibody detection reagents were prepared to carry out an experiment on the detection of an anti-HCV antibody by EIA and ECL using, as an antigen, an octamer of a peptide having the amino acid sequence specified in SEQ ID NO: 2.

As a result, the anti-HCV antibody could be detected in 11 specimen samples out of the 22 specimen samples. The measurements on the 11 specimen samples from which the anti-HCV antibody could be detected are given in Table 3. Thus, serum specimen samples from which the anti-HCV antibody has not been detected by the conventional anti-HCV antibody detection reagents can be judged to be positive by the detection reagent of the present invention, confirming that the reagent of the present invention has a superior anti-HCV antibody detection accuracy.

**Table 3**

| Sample | RNA | Reagent A | Reagent B (CI) | Reagent C (CI) | Reagent D | Reagent of invention (EIA, OD₄₀₅) | Reagent of invention (ECL, counts) |
|---|---|---|---|---|---|---|---|
| A-11 | + | - | 0.44 | 0.25 | NT | 0.51 | 295009 |
| III-13 | + | - | 0.16 | 0.07 | - | 1.23 | 608951 |
| 27 | + | - | 0.20 | 0.07 | - | 0.37 | 703138 |
| 52 | + | - | 0.14 | 0.04 | - | 0 38 | 418334 |
| 102 | + | - | 0.15 | 0.07 | - | 0.28 | 70050 |
| 113 | + | - | 0.16 | 0.04 | - | 0.22 | 350701 |
| 128 | + | - | 0.17 | 0.15 | - | 1.55 | 9320443 |
| 134 | + | - | 0.19 | 0.07 | - | 0.77 | 849779 |
| 142 | + | - | 0.15 | 0.07 | - | 0.38 | 123119 |
| 144 | + | - | 0.16 | 0.04 | - | 0.71 | 1376967 |
| 145 | + | - | 0.13 | 0.04 | - | 0.69 | 248866 |
| PC | + | - | 5.02 | 13.93 | NT | 1.10 | 874830 |
| NHS-1 | + | - | 0.17 | 0.04 | NT | 0.03 | 5938 |
| NHS-2 | + | - | 0.14 | 0.63 | NT | 0.05 | NT |
| NHS-3 | + | - | 0.20 | 0.04 | NT | 0.04 | NT |
| NHS-4 | + | - | 0.23 | 0.07 | NT | 0.03 | NT |
| RNA +: HCV-RNA positive, Reagent A: HCV PHA Dainabot, Reagent B: Immucheck HCV Ab, Reagent C: Synpep HCV-EIA, Reagent D: RIBA II, EIA: Enzyme Immunoassay, ECL: Electrochemiluminescence, Reagent of invention: Octamer of the peptide specified in SEQ ID NO: 2 was used as the antigen, NT: not tested, OD₄₀₅: absorbance at 405 nm, CI: Cut off index, PC: positive standard serum, and NHS: normal human serum. | | | | | | | |

### Example 8: Monitoring of the effect of interferon therapy

In interferon therapy for patients suffering from hepatitis C, five cases where the interferon therapy was significantly effective and another five cases where the interferon therapy was ineffective were checked for the change in plasma anti-HCV antibody level by ECL using, as an antigen, an octamer of a peptide having the amino acid sequence specified in SEQ ID NO: 12.

The results are given in Figs. 2a to 2d. As compared with an anti-HCV antibody detection reagent Chemo-Sero EIA "JCC-2" (Ortho Pharmaceutical) used as a control, the reagent of the invention detected early the lowering of the antibody titer for the cases where the interferon therapy was effective (see Fig. 2c), demonstrating that the reagent of the present invention can judge the therapeutic effect in an earlier stage. On the other hand, for "JCC-2", this correlation was not very clear.

Sequence Listing

(2) INFORMATION FOR SEQ ID NO: 1:

(i) SEQUENCE CHARACTERISTICS:

(A) LENGTH: 12 amino acids

(B) TYPE: amino acid

(D) TOPOLOGY: linear

(ii) MOLECULE TYPE: peptide

(xi) SEQUENCE DESCRIPTION: SEQ ID NO: 1:

Ala His Gly Ile Glu Pro Asn Ile Arg Thr Gly Val
1               5                   10

(2) INFORMATION FOR SEQ ID NO: 2:

(i) SEQUENCE CHARACTERISTICS:

(A) LENGTH: 13 amino acids

(B) TYPE: amino acid

(D) TOPOLOGY: linear

(ii) MOLECULE TYPE: peptide

(xi) SEQUENCE DESCRIPTION: SEQ ID NO: 2:

Ala His Gly Ile Glu Pro Asn Ile Arg Thr Gly Val Arg
1               5                   10

(2) INFORMATION FOR SEQ ID NO: 3:

(i) SEQUENCE CHARACTERISTICS:

(A) LENGTH: 13 amino acids

(B) TYPE: amino acid

(D) TOPOLOGY: linear

(ii) MOLECULE TYPE: peptide

(xi) SEQUENCE DESCRIPTION: SEQ ID NO: 3:

Ala His Gly Ile Asp Pro Asn Ile Arg Thr Gly Val Arg
1               5                   10

(2) INFORMATION FOR SEQ ID NO: 4:

(i) SEQUENCE CHARACTERISTICS:

(A) LENGTH: 13 amino acids

(B) TYPE: amino acid

(D) TOPOLOGY: linear

(ii) MOLECULE TYPE: peptide

(xi) SEQUENCE DESCRIPTION: SEQ ID NO: 4:

Ala His Gly Ile Asn Pro Asn Ile Arg Thr Gly Val Arg
1               5                   10

(2) INFORMATION FOR SEQ ID NO: 5:

(i) SEQUENCE CHARACTERISTICS:

(A) LENGTH: 13 amino acids

(B) TYPE: amino acid

(D) TOPOLOGY: linear

(ii) MOLECULE TYPE: peptide

(xi) SEQUENCE DESCRIPTION: SEQ ID NO: 5:

Ala His Gly Ile Asp Pro Asn Thr Arg Thr Gly Val Arg
1               5                   10

(2) INFORMATION FOR SEQ ID NO: 6:

(i) SEQUENCE CHARACTERISTICS:

(A) LENGTH: 13 amino acids

(B) TYPE: amino acid

(D) TOPOLOGY: linear

(ii) MOLECULE TYPE: peptide

(xi) SEQUENCE DESCRIPTION: SEQ ID NO: 6:

Ala His Gly Ile Asp Pro Asn Val Arg Thr Gly Val Arg
1               5                   10

(2) INFORMATION FOR SEQ ID NO: 7:

(i) SEQUENCE CHARACTERISTICS:

(A) LENGTH: 13 amino acids

(B) TYPE: amino acid

(D) TOPOLOGY: linear

(ii) MOLECULE TYPE: peptide

(xi) SEQUENCE DESCRIPTION: SEQ ID NO: 7:

Ala His Gly Ile Asp Pro Asn Leu Arg Thr Gly Val Arg
1               5                   10

(2) INFORMATION FOR SEQ ID NO: 8:

(i) SEQUENCE CHARACTERISTICS:

(A) LENGTH: 13 amino acids

(B) TYPE: amino acid

(D) TOPOLOGY: linear

(ii) MOLECULE TYPE: peptide

(xi) SEQUENCE DESCRIPTION: SEQ ID NO: 8:

Ala His Gly Ile Glu Pro Asn Thr Arg Thr Gly Val Arg
1               5                   10

(2) INFORMATION FOR SEQ ID NO: 9:

(i) SEQUENCE CHARACTERISTICS:

(A) LENGTH: 13 amino acids

(B) TYPE: amino acid

(D) TOPOLOGY: linear

(ii) MOLECULE TYPE: peptide

(xi) SEQUENCE DESCRIPTION: SEQ ID NO: 9:

Ala His Gly Ile Glu Pro Asn Val Arg Thr Gly Val Arg
1               5                   10

(2) INFORMATION FOR SEQ ID NO: 10:

(i) SEQUENCE CHARACTERISTICS:

(A) LENGTH: 13 amino acids

(B) TYPE: amino acid

(D) TOPOLOGY: linear

(ii) MOLECULE TYPE: peptide

(xi) SEQUENCE DESCRIPTION: SEQ ID NO: 10:

Ala His Gly Ile Glu Pro Asn Leu Arg Thr Gly Val Arg
1               5                   10

(2) INFORMATION FOR SEQ ID NO: 11:

(i) SEQUENCE CHARACTERISTICS:

(A) LENGTH: 15 amino acids

(B) TYPE: amino acid

(D) TOPOLOGY: linear

(ii) MOLECULE TYPE: peptide

(xi) SEQUENCE DESCRIPTION: SEQ ID NO: 11:

Asn Pro Lys Pro Gln Arg Lys Thr Lys Arg Asn Thr Asn
1               5                   10

Arg Arg
    15

(2) INFORMATION FOR SEQ ID NO: 12:

(i) SEQUENCE CHARACTERISTICS:

(A) LENGTH: 17 amino acids

(B) TYPE: amino acid

(D) TOPOLOGY: linear

(ii) MOLECULE TYPE: peptide

(xi) SEQUENCE DESCRIPTION: SEQ ID NO: 12:

Pro Asp Lys Glu Ile Leu Tyr Glu Ala Phe Asp Glu Met
1               5                   10

Glu Glu Cys Ala
    15

(2) INFORMATION FOR SEQ ID NO: 13:

(i) SEQUENCE CHARACTERISTICS:

(A) LENGTH: 15 amino acids

(B) TYPE: amino acid

(D) TOPOLOGY: linear

(ii) MOLECULE TYPE: peptide

(xi) SEQUENCE DESCRIPTION: SEQ ID NO: 13:

Pro Asp Arg Glu Val Leu Tyr Gln Glu Phe Asp Glu Met
1               5                   10

Glu Glu
    15

SEQUENCE LISTING

(1) GENERAL INFORMATION:

(i) APPLICANT:
(A) NAME: Eisai Co., Ltd.
(B) STREET: 6-10, Koishikawa 4-chome
(C) CITY: Bunkyo-ku, Tokyo
(E) COUNTRY: Japan
(F) POSTAL CODE (ZIP): none

(A) NAME: Terukatsu ARIMA
(B) STREET: 5-1-403, Hiranocho
(C) CITY: Kagoshima-shi, Kagoshima
(E) COUNTRY: Japan
(F) POSTAL CODE (ZIP): none

(ii) TITLE OF INVENTION: Multiple antigenic peptide comprising at
least two hepatitis C virus-associated peptides

(iii) NUMBER OF SEQUENCES: 13

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 96105063.0

(2) INFORMATION FOR SEQ ID NO: 1:

(i) SEQUENCE CHARACTERISTICS:
(A) LENGTH: 12 amino acids
(B) TYPE: amino acid

(C) STRANDEDNESS:
(D) TOPOLOGY: linear

(ii) MOLECULE TYPE: peptide

(xi) SEQUENCE DESCRIPTION: SEQ ID NO: 1:

Ala His Gly Ile Glu Pro Asn Ile Arg Thr Gly Val
1               5                   10

(2) INFORMATION FOR SEQ ID NO: 2:

(i) SEQUENCE CHARACTERISTICS:
(A) LENGTH: 13 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 2:

Ala His Gly Ile Glu Pro Asn Ile Arg Thr Gly Val Arg
1               5                   10

(2) INFORMATION FOR SEQ ID NO: 3:

(i) SEQUENCE CHARACTERISTICS:
(A) LENGTH: 13 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 3:

Ala His Gly Ile Asp Pro Asn Ile Arg Thr Gly Val Arg
1               5                   10

(2) INFORMATION FOR SEQ ID NO: 4:

(i) SEQUENCE CHARACTERISTICS:
(A) LENGTH: 13 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 4:

Ala His Gly Ile Asn Pro Asn Ile Arg Thr Gly Val Arg
1               5                   10

(2) INFORMATION FOR SEQ ID NO: 5:

(i) SEQUENCE CHARACTERISTICS:
(A) LENGTH: 13 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 5:

Ala His Gly Ile Asp Pro Asn Thr Arg Thr Gly Val Arg
1               5                   10

(2) INFORMATION FOR SEQ ID NO: 6:

(i) SEQUENCE CHARACTERISTICS:

(A) LENGTH: 13 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 6:

Ala His Gly Ile Asp Pro Asn Val Arg Thr Gly Val Arg
1               5                   10

(2) INFORMATION FOR SEQ ID NO: 7:

(i) SEQUENCE CHARACTERISTICS:
(A) LENGTH: 13 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 7:

Ala His Gly Ile Asp Pro Asn Leu Arg Thr Gly Val Arg
1               5                   10

(2) INFORMATION FOR SEQ ID NO: 8:

(i) SEQUENCE CHARACTERISTICS:
(A) LENGTH: 13 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 8:

Ala His Gly Ile Glu Pro Asn Thr Arg Thr Gly Val Arg
1               5                   10

(2) INFORMATION FOR SEQ ID NO: 9:

(i) SEQUENCE CHARACTERISTICS:
(A) LENGTH: 13 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 9:

Ala His Gly Ile Glu Pro Asn Val Arg Thr Gly Val Arg
1               5                   10

(2) INFORMATION FOR SEQ ID NO: 10:

(i) SEQUENCE CHARACTERISTICS:
(A) LENGTH: 13 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 10:

Ala His Gly Ile Glu Pro Asn Leu Arg Thr Gly Val Arg
1               5                   10

(2) INFORMATION FOR SEQ ID NO: 11:

(i) SEQUENCE CHARACTERISTICS:
(A) LENGTH: 15 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 11:

Asn Pro Lys Pro Gln Arg Lys Thr Lys Arg Asn Thr Asn Arg Arg
1               5                   10                  15

(2) INFORMATION FOR SEQ ID NO: 12:

(i) SEQUENCE CHARACTERISTICS:
(A) LENGTH: 17 amino acids
(B) TYPE: amino acid
(C) STRANDEDNESS:
(D) TOPOLOGY: linear

(ii) MOLECULE TYPE: peptide

(xi) SEQUENCE DESCRIPTION: SEQ ID NO: 12:

Pro Asp Lys Glu Ile Leu Tyr Glu Ala Phe Asp Glu Met Glu Glu Cys
1               5                   10                  15

Ala

(2) INFORMATION FOR SEQ ID NO: 13:

(i) SEQUENCE CHARACTERISTICS:
(A) LENGTH: 15 amino acids
(B) TYPE: amino acid
(C) STRANDEDNESS:

(D) TOPOLOGY: linear

(ii) MOLECULE TYPE: peptide

(xi) SEQUENCE DESCRIPTION: SEQ ID NO: 13:

Pro Asp Arg Glu Val Leu Tyr Gln Glu Phe Asp Glu Met Glu Glu
1               5                   10                  15

## Claims

1. A multiple antigenic peptide which comprises at least two hepatitis C virus-associated peptides each comprising at least eight consecutive amino acid residues in an amino acid sequence of a protein constituting a hepatitis C virus.

2. The multiple antigenic peptide according to Claim 1, which further comprises a core matrix.

3. The multiple antigenic peptide according to Claim 1, which comprises two to eight hepatitis C virus-associated peptides.

4. The multiple antigenic peptide according to Claim 1, which comprises eight hepatitis C virus-associated peptides.

5. The multiple antigenic peptide according to Claim 1, wherein the hepatitis C virus-associated peptide comprises twelve consecutive amino acid residues described in Sequence ID NO: 1, or an immunochemically eqivalent of the twelve consecutive amino acid residues.

6. The multiple antigenic peptide according to Claim 1, wherein the hepatitis C virus-associated peptide comprises thirteen consecutive amino acid residues described in Sequence ID NO: 2, 3, 4, 5, 6, 7, 8, 9 or 10, an immunochemically eqivalent of the thirteen consecutive amino acid residues, fifteen consecutive amino acid residues described in Sequence ID NO: 11 or 13, an immunochemically eqivalent of the fifteen consecutive amino acid residues, seventeen consecutive amino acid residues described in Sequence ID NO: 12, or an immunochemically eqivalent of the seventeen consecutive amino acid residues.

7. The multiple antigenic peptide according to Claim 6, wherein the hepatitis C virus-associated peptide comprises thirteen consecutive amino acid residues described in Sequence ID NO: 11 or 13, an immunochemically eqivalent of the thirteen consecutive amino acid residues, seventeen consecutive amino acid residues described in Sequence ID NO: 12, or an immunochemically eqivalent of the seventeen consecutive amino acid residues.

8. A reagent for assaying an anti-hepatitis C virus antibody, which comprises, as an antigen for detecting the anti-hepatitis C virus antibody, at least one multiple antigenic peptide of Claim 1.

9. The reagent for assaying an anti-hepatitis C virus antibody according to Claim 8, wherein the multiple antigenic peptide comprises two to eight hepatitis C virus-associated peptides.

10. The reagent for assaying an anti-hepatitis C virus antibody according to Claim 8, which comprises, as an antigen for detecting the anti-hepatitis C virus antibody, at least one member selected from the group consisting of multiple antigenic peptides each comprising twelve consecutive amino acid residues described in Sequence ID NO: 1, an immunochemically eqivalent of the twelve consecutive amino acid residues, thirteen consecutive amino acid residues described in Sequence ID NO: 2, 3, 4, 5, 6, 7, 8, 9 or 10, an immunochemically eqivalent of the thirteen consecutive amino acid residues, fifteen consecutive amino acid residues described in Sequence ID NO: 11 or 13, an immunochemically eqivalent of the fifteen consecutive amino acid residues, seventeen consecutive amino acid residues described in Sequence ID NO: 12, or an immunochemically eqivalent of the seventeen consecutive amino acid residues.

11. A method for immunochemically assaying an anti-hepatitis C virus antibody, which comprises the steps of:
reacting a specimen sample with a multiple antigenic peptide of Claim 1,
reacting the reaction product with a labelled anti-human immunoglobulin antibody, and
determining the amount of the labelled anti-human immunoglobulin antibody bound to the reaction product.

12. The method for immunochemically assaying an anti-hepatitis C virus antibody according to Claim 11, wherein the specimen sample is a human biological sample.

13. The method for immunochemically assaying an anti-hepatitis C virus antibody according to Claim 11, wherein the multiple antigenic peptide has been immobilized on a carrier.

14. The method for immunochemically assaying an anti-hepatitis C virus antibody according to Claim 13, wherein the carrier is a solid phase.
